# NEW EUROPEAN PATENT SPECIFICATION

(11) **EP 0 850 295 B2**
(45) Date of publication and mention of the opposition decision: **30.11.2011**
(45) Mention of the grant of the patent: 21.05.2003
(21) Application number: 96928351.4
(22) Date of filing: 03.09.1996
(51) Int. Cl.: C11D 3/386, D06M 16/00, C12N 9/42

(54) **PREVENTION OF BACK-STAINING IN STONE WASHING**
VERHÜTUNG DES VERSCHMIERENS BEIM STONE-WASHING
PREVENTION DU MACULAGE D'ENVERS LORS DU LAVAGE A LA PIERRE

(30) Priority: 08.09.1995 DK 99395
(43) Date of publication of application: 01.07.1998
(73) Proprietor: Novozymes A/S, 2880 Bagsvaerd (DK)
(72) Inventor: ONISHI, Masahiro, Chiba-city 267 (JP); FICH, Merete, DK-2920 Charlottenlund (DK); TOFT, Annette, Hanne, DK-2880 Bagsvaerd (DK); SCHÜLEIN, Martin, DK-2100 Koebenhavn OE (DK)
(74) Representative: Shenker, Paul Dhan
(86) International application number: PCT/DK1996/000364
(87) International publication number: WO 1997/009410

(56) References cited:
- WO-A1-94/07983
- WO-A1-94/29426
- WO-A1-95/24471
- WO-A1-95//02675
- CHEMICAL ABSTRACTS, Volume 121, No. 18, 31 October 1994, (Columbus, Ohio, USA), KLAHORST, SUANNE et al., "Optimizing the Use of Cellulases for Denim Finishing", page 120, Abstract No. 207550s, Book Pap.; & INT. CONF. EXHIB., 1992, 243-249. XP008075682
- BIOCHEM. J., Volume 280, 1991, BERNARD HENRISSAT, "A Classification of Glycosyl Hydrolases Based on Amino Acid Squence Similarities", pages 309-316. XP002050142
- BIOCHEM. J., Volume 293, 1993, BERNARD HENRISSAT et al., "New Families in the Classification of Glycosyl Hydrolases Based on Amino Acid Sequence Similarities", pages 781-788. XP000869530
- KIEYWEGT ET AL: 'Structure of T. reesei Endoglucanase I' J. MOL. BIOL. vol. 272, 1997, pages 383 - 397
- DOMINGUEZ ET AL: 'Spontaneous aggregation of Endogluconase I from Trichoderma reesei QM9414' BIOTECHNOL APP. BIOCHEM. vol. 15, 1992, pages 236 - 246
- OKADA ET AL: 'Molecular characterisation and heterologous expression of the gene encoding a low-molecular-mass endoglucanase from Trichoderma reesei QM9414' APP. ENVIRON. MICROBIOL. vol. 64, 1998, pages 555 - 563

## Description

### TECHNICAL FIELD

This invention relates to a method of forming localized variation of color density in the surface of a dyed cellulosic fabric, and to a composition for use in the method.

### BACKGROUND ART

In the manufacture of garments from dyed cellulosic fabric, e.g., blue jeans from indigo-dyed denim, it is common to treat the denim so as to provide a "stone-washed" look (localized abrasion of the color in the denim surface). This can be achieved by agitating the denim in an aqueous medium containing a mechanical abrasion agent such as pumice, an abrading cellulase or a combination of these. It is preferred to run the process near neutral pH, so it is preferred to use a cellulase with high activity in this pH range. In the past, cellulase preparations were generally produced by cultivation of naturally occurring microorganisms, and such preparations invariably contained a mixture of many different cellulase components. A process using such a mixed cellulase preparation is described in US 4,832,864 (to Ecolab).

The rapid advances in recombinant DNA techniques have made it possible to produce single-component enzymes in high yield, and processes using single-component cellulases have therefore become more interesting. Thus, WO 91/17243 and WO 95/09225 (Novo Nordisk) describe a process using a single-component endoglucanase denoted EG V with a molecular weight of ∼43 kD derived from *Humicola insolens* strain DSM 1800 with optimum activity near neutral pH. WO 94/21801 (Genencor) describes the use in "stone washing" of a single-component cellulase called EG III derived from *Trichoderma longibrachiatum* which is reported to have a pH optimum of 5.5-6.0 and to retain significant activity at alkaline pH. WO 95/16782 (Genencor International) suggests the use of other single-component cellulases derived from *Trichoderma* in "stone washing", but these cellulases are acidic and have virtually no activity at neutral pH.

A general problem in the known "stone washing" methods is that of back-staining, i.e. a phenomenon whereby dye already removed by abrasion deposits on parts of the fabric or garment so as to even out the desired variation of color density or to discolor any light-colored parts of the garment

### STATEMENT OF THE INVENTION

We have found that, surprisingly, the addition of a certain type of cellulase (hereinafter denoted as the first component) reduces back-staining. The cellulase in question has no significant abrading effect in itself.

Accordingly, the invention provides a method of forming localized variation of color density in the surface of a dyed cellulosic fabric according to claim 1.

### DEFINITIONS

In this specification with claims, the following definitions apply:

The term "cellulase" denotes an enzyme that contributes to the hydrolysis of cellulose, such a cellobiohydrolase (Enzyme Nomenclature E.C. 3.2.1.91), an endoglucanase (hereinafter abbreviated as "EG", E.C. 3.2.1.4), or a b-glucosidase (E.C. 3.2.1.21).

Cellulases are classified into families on the basis of amino-acid sequence similarities according to the classification system described in Henrissat, B. et al.: Biochem. J., (1991), 280, p. 309-16, and Henrissat, B. et al.: Biochem. J., (1993), 293, p. 781-788.

The cellulases used in this invention are preferably single components, i.e. the aqueous medium used in the invention should be free of other cellulase components than those specified. Single component enzymes can be prepared economically by recombinant DNA technology, i.e. they can be produced by cloning of a DNA sequence encoding the single component, subsequently transforming a suitable host cell with the DNA sequence and expressing the component in the host.

Accordingly, the DNA sequence encoding a useful cellulase may be isolated by a general method involving
- cloning, in suitable vectors, a DNA library e.g. from one of the microorganisms indicated later in this specification,
- transforming suitable yeast host cells with said vectors,
- culturing the host cells under suitable conditions to express any enzyme of interest encoded by a clone in the DNA library,
- screening for positive clones by determining any cellulase activity of the enzyme produced by such clones, and
- isolating the enzyme encoding DNA from such clones.

The general method is further disclosed in WO 94/14953 (Novo Nordisk).

The DNA sequence coding for a useful cellulase may for instance be isolated by screening a cDNA library of the microorganism in question and selecting for clones expressing the appropriate enzyme activity (i.e. cellulase activity).

Alternatively, the DNA coding for a useful cellulase may, in accordance with well-known procedures, conveniently be isolated from DNA from a suitable source, by use of synthetic oligonucleotide probes prepared on the basis of a known DNA sequence.

The DNA sequence may subsequently be inserted into a recombinant expression vector. This may be any vector which may conveniently be subjected to recombinant DNA procedures, and the choice of vector will often depend on the host cell into which it is to be introduced. Thus, the vector may be an autonomously replicating vector, i.e. a vector which exists as an extrachromosomal entity, the replication of which is independent of chromosomal replication, e.g. a plasmid. Alternatively, the vector may be one which, when introduced into a host cell, is integrated into the host cell genome and replicated together with the chromosome(s) into which it has been integrated.

In the vector, the DNA sequence encoding the cellulase should be operably connected to a suitable promoter and terminator sequence. The promoter may be any DNA sequence which shows transcriptional activity in the host cell of choice and may be derived from genes encoding proteins either homologous or heterologous to the host cell. The procedures used to ligate the DNA sequences coding for the cellulase, the promoter and the terminator, respectively, and to insert them into suitable vectors are well known to persons skilled in the art (cf., for instance, Sambrook et al., Molecular Cloning. A Laboratory Manual, Cold Spring Harbor, NY, 1989).

The host cell which is transformed with the DNA sequence is preferably a eukaryotic cell, in particular a fungal cell such as a yeast or filamentous fungal cell. In particular, the cell may belong to a species of *Aspergillus* or *Trichoderma*, most preferably *Aspergillus oryzae* or *Aspergillus niger.* Fungal cells may be transformed by a process involving protoplast formation and transformation of the protoplast followed by regeneration of the cell wall in a manner known per se. The use of *Aspergillus* as a host microorganism is described in EP 238 023 (Novo Nordisk A/S). The host cell may also be a yeast cell, e.g. a strain of *Saccharomyces*, in particular *Saccharomyces cerevisiae, Saccharomyces kluyveri* or *Saccharomyces uvarum*, a strain of *Schizosaccharomyces sp.,* such as *Schizosaccharomyces pombe,* a strain of *Hansenula sp., Pichia sp., Yarrowia sp.* such as *Yarrowia lipolytica,* or *Kluyveromyces sp*. such as *Kluyveromyces lactis*.

### DETAILED DESCRIPTION OF THE INVENTION

### Dyed cellulosic fabric

The method of the invention may be applied to any type of dyed cellulosic fabric where it is desired to form localized variation of color density in the surface. An example of particular commercial interest is denim, particularly indigo-dyed denim for use in blue jeans etc.

The fabric may be treated in the form of unsewn fabric or a sewn garment made of such fabric. It is of particular interest to apply the method of the invention to new, clean fabric or garment.

### Component 1

The first component is endoglucanase EG I derived from *Humicola insolens* DSM 1800. It is present in an effective amount for preventing back-staining, typically 0.05-5 mg/l (as pure enzyme protein), particularly 0.1-0.5 mg/l; typically corresponding to an activity of 10-1000 ECU/I, particularly 100-1000 ECU/I; or an activity of 0.5-100 ECU/g of fabric.

### Component 2

The second is endoglucanase EG V derived from *Humicola insolens* strain DSM 1800. A preferred embodiment of the invention additionally comprises a mechanical abrading agent.

Examples of mechanical abrading agents are pumice, heat expanded perlite and abrading elements (e.g. abrading balls).

The second component is present in an effective amount for abrasion to form localized variation of color density. If the second component is an abrading cellulase, it is typically present in an amount of 0.05-5 mg/l (as pure enzyme protein), particularly 0.1-0.5 mg/l; typically corresponding to an activity of 10-1000 ECU/I, particularly 100-1000 ECU/I; or an activity of 0.1-100 ECU/g of fabric, particularly 0.5-10 ECU/g.

### Method conditions

The method of the invention may be carried out at conventional conditions in a washing machine conventionally used for stone-washing (e.g. a washer-extractor). Typical conditions are a temperature of 40-60°C and a fabric : liquor ratio from 1:3 to 1:20 for 15 minutes to 2 hours. Optionally, conventional additives may be used, e.g. a buffer, a surfactant (anionic and/or non-ionic) and/or a polymer (such as PVP, polyacrylate and polyacrylamide).

### Assay for cellulase activity

The cellulase endo-activity is determined by the reduction of viscosity of CMC (carboxy-methyl cellulose) in a vibration viscosimeter. 1 ECU (endo-cellulase unit) is the amount of activity which causes a 10-fold reduction of viscosity when incubated with 1 ml of a solution of 34.0 g/L of CMC (trade name Aqualon 7LFD) in 0.1 M phosphate buffer (pH 7.5), 40°C for 30 minutes.

### Assay for hydrolysis of PNP-Cel

The ability of a cellulase to hydrolyze p-nitrophenyl-b-1,4-cellobioside (PNP-Cel) is determined by steady-state kinetic, direct detection of the yellow color of the product p-nitrophenol (PNP) by absorption at 405 nm. The assay conditions are 37°C, pH 7.5 (0.1 M phosphate buffer). The hydrolysis rate (in micromol of PNP per minute) is compared to the cellulase activity (ECU), and the result is expressed as micromol PNP per minute per ECU.

### EXAMPLES

### Example 1

Indigo-dyed denim was treated together with white cotton swatches using various combinations of cellulase, as follows:

| | |
|---|---|
| pH | 7 (phosphate buffer in tap water) |
| Temperature | 55°C |
| Equipment | Launderometer (150 ml containers) |
| Component 1 | EG I derived from *Humicola insolens* DSM 1800 0-2.3 ECU/ml as indicated below |
| Component 2 | EG V derived from *Humicola insolens* DSM 1800 0 or 0.27 ECU/ml |
| Denim | 5 g/container |
| White cotton | 2 swatches/container |
| Time | 2 hours |

After the treatment, lint was collected and measured as an expression of the abrading action of the cellulase(s). The remission of the white swatches after the treatment was measured (D R at 680 nm, relative to an experiment without any cellulase) and taken as an expression of back-staining. Results:

| | Component 1 ECU/ml | Component 2 ECU/ml | Abrasion (mg lint) | Back-staining reduction (D R) |
|---|---|---|---|---|
| Reference | 0 | 0 | - | 0 |
| | 0 | 0.27 | 33 | -3.8 |
| Invention | 0.115 | 0.27 | 42 | -0.7 |
| | 0.23 | 0.27 | 36 | 3.0 |
| | 1.15 | 0.27 | 38 | 8.2 |
| | 2.3 | 0.27 | 62 | 10.4 |

Good abrasion was obtained with the component 2 cellulase. The addition of the component 1 cellulase significantly reduced the back-staining.

### Example 2

The following cellulases were tested at the same conditions as in Example 1:

| | |
|---|---|
| Component 1 | EG I derived from *Humicola insolens* DSM 1800 0,0.67 or 1.33 ECU/ml |
| Component 2 | EG V derived from *Humicola insolens* DSM 1800, 0.7 ECU/ml |

Abrasion was evaluated by measuring the amount of lint after each treatment. Good abrasion was found in each experiment, with a slight increase by addition of EG I.

Back-staining inhibition was determined from the increase of absorbance of the filtrate at 680 nm and from the increase of remission of the white fabric at 420 nm. The results showed that essentially the same back-staining inhibition was obtained with EG I.

## Claims

1. A method of forming localized variation of color density in the surface of a dyed cellulosic fabric, comprising agitating the fabric in an aqueous medium having a pH in the range 6.5-9 and containing:
a first component which is endoglucanase EG I derived from *H. insolens* strain DSM 1800
and a second component which is endoglucanase EG V derived from *H. insolens* strain DSM 1800
wherein each cellulase displays at least 30% of its maximum activity at pH 7, and wherein essentially no cellulase other than the specified first and second component is present.

2. The method of claim 1 wherein the fabric is indigo dyed denim.

3. The method of any of claims 1-2 wherein the first component is present in an amount of 0.1-0.5 mg/l or at a concentration of 100-1000 ECU/l.

4. The method of any of claims 1-3 wherein the second component comprises additionally a mechanical abrading agent.

## Patentansprüche

1. Verfahren zum Bilden einer lokalisierten Variation der Farbdichte in der Oberfläche eines gefärbten Zellulosegewebes, umfassend ein Hin- und Herbewegen des Gewebes in einem wässrigen Medium mit einem pH in dem Bereich 6,5-9 und enthaltend:
eine erste Komponente, welche Endoglucanase EG I ist, abgeleitet von dem *H. insolens*-Stamm DSM 1800,
und eine zweite Komponente, welche Endoglucanase EG V ist, abgeleitet von dem *H. insolens*-Stamm DSM 1800,
wobei jede Zellulase mindestens 30 % ihrer maximalen Aktivität bei pH 7 zeigt, und wobei im wesentlichen keine Zellulase außer der spezifizierten ersten und zweiten Komponente vorhanden ist.

2. Verfahren nach Anspruch 1, in welchem das Gewebe ein indigogefärbter Jeansstoff (Denim) ist.

3. Verfahren nach einem beliebigen der Ansprüche 1-2, in welchem die erste Komponente in einer Menge von 0,1-0,5 mg/l oder ein einer Konzentration von 100-1.000 ECU/l vorhanden ist.

4. Verfahren nach einem beliebigen der Ansprüche 1-3, in welchem die zweite Komponente zusätzlich einen mechanisch abreibenden Wirkstoff umfasst.

## Revendications

1. Un procédé de formation d'une variation localisée de densité de couleur à la surface d'un tissu cellulosique teint, comprenant l'agitation du tissu dans un milieu aqueux ayant un pH compris dans la plage 6,5 à 9 et contenant :
un premier composant qui est une endoglucanase EG I dérivée d'une souche *H. insolens* DSM 1800,
et un second composant qui est une endoglucanase EG V dérivée d'une souche *H. insolens* DSM 1800,
dans lequel chaque cellulase affiche au moins 30% de son activité maximale à pH7, et dans lequel n'est essentiellement présente aucune cellulase autre que les premier et second composants spécifiés.

2. Le procédé de la revendication 1, dans lequel le tissu est du denim teint indigo.

3. Le procédé de l'une des revendications 1 à 2, dans lequel le premier composant est présent en une quantité de 0,1 à 0,5 mg/l où à une concentration de 100 à 1000 ECU/l.

4. Le procédé de l'une des revendications 1 à 3, dans lequel le second composant comprend en outre un agent d'abrasion mécanique.


### SEQUENCE LISTING

(1) GENERAL INFORMATION:
   (i) APPLICANT:
      (A) NAME: Novo Nordisk A/S
      (B) STREET: Novo Alle
      (C) CITY: Bagsvaerd
      (E) COUNTRY: Denmark
      (F) POSTAL CODE (ZIP): DK-2880
      (G) TELEPHONE: +45-4444-8888
      (H) TELEFAX: +45-4449-3256
   (ii) TITLE OF INVENTION: Prevention of back-staining in stone washing
   (iii) NUMBER OF SEQUENCES: 1
   (iv) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: PatentIn Release #1.0, Version #1.30 (EPO)
(2) INFORMATION FOR SEQ ID NO: 1:
   (i) SEQUENCE CHARACTERISTICS:
      (A) LENGTH: 551 amino acids
      (B) TYPE: amino acid
      (C) STRANDEDNESS:
      (D) TOPOLOGY: linear (ii) MOLECULE TYPE: protein
   (vi) ORIGINAL SOURCE:
      (A) ORGANISM: Bacillus lautus
      (B) STRAIN: NCIMB 40250
   (xi) SEQUENCE DESCRIPTION: SEQ ID NO: 1:
   Ala Pro Ala Val Pro Phe Gly Gln Leu Lys Val Gln Gly Asn Gln Leu
   1               5                   10                  15
   Val Gly Gln Ser Gly Gln Ala Val Gln Leu Val Gly Met Ser Ser His
               20                  25                  30
   Gly Leu Gln Trp Tyr Gly Asn Phe Val Asn Lys Ser Ser Leu Gln Trp
           35                  40                  45
   Met Arg Asp Asn Trp Gly Ile Asn Val Phe Arg Ala Ala Met Tyr Thr
       50                  55                  60
   Ala Glu Asp Gly Tyr Ile Thr Asp Pro Ser Val Lys Asn Lys Val Lys
   65                  70                  75                  80
   Glu Ala Val Gln Ala Ser Ile Asp Leu Gly Leu Tyr Val Ile Ile Asp
                   85                  90                  95
   Trp His Ile Leu Ser Asp Gly Asn Pro Asn Thr Tyr Lys Ala Gln Ser
               100                 105                 110
   Lys Ala Phe Phe Gln Glu Met Ala Thr Leu Tyr Gly Asn Thr Pro Asn
           115                 120                 125
   Val Ile Tyr Glu Ile Ala Asn Glu Pro Asn Gly Asn Val Ser Trp Ala
       130                 135                 140
   Asp Val Lys Ser Tyr Ala Glu Glu Val Ile Thr Ala Ile Arg Ala Ile
   145                 150                 155                 160
   Asp Pro Asp Gly Val Val Ile Val Gly Ser Pro Thr Trp Ser Gln Asp
                   165                 170                 175
   Ile His Leu Ala Ala Asp Asn Pro Val Ser His Ser Asn Val Met Tyr
               180                 185                 190
   Ala Leu His Phe Tyr Ser Gly Thr His Gly Gln Phe Leu Arg Asp Arg
           195                 200                 205
   Ile Thr Tyr Ala Met Asn Lys Gly Ala Ala Ile Phe Val Thr Glu Trp
       210                 215                 220
   Gly Thr Ser Asp Ala Ser Gly Asn Gly Gly Pro Tyr Phe Pro Gln Ser
   225                 230                 235                 240
   Lys Glu Trp Ile Asp Phe Leu Asn Ala Arg Lys Ile Ser Trp Val Asn
                   245                 250                 255
   Trp Ser Leu Ala Asp Lys Val Glu Thr Ser Ala Ala Leu Met Pro Gly
               260                 265                 270
   Ala Ser Pro Thr Gly Gly Trp Thr Asp Ala Gln Leu Ser Glu Ser Gly
           275                 280                 285
   Lys Trp Val Arg Asp Gln Ile Arg Gln Ala Thr Gly Gly Gly Ser Gly
       290                 295                 300
   Asn Pro Thr Ala Pro Ala Ala Pro Thr Asn Leu Ser Ala Thr Ala Gly
   305                 310                 315                 320
   Asn Ala Gln Val Ser Leu Thr Trp Asn Ala Val Ser Gly Ala Thr Ser
                   325                 330                 335
   Tyr Thr Val Lys Arg Ala Thr Thr Ser Gly Gly Pro Tyr Thr Asn Val
               340                 345                 350
   Ala Thr Gly Val Thr Ala Thr Ser Tyr Thr Asn Thr Gly Leu Thr Asn
           355                 360                 365
   Gly Thr Thr Tyr Tyr Tyr Val Val Ser Ala Ser Asn Ser Ala Gly Ser
       370                 375                 380
   Ser Ala Asn Ser Ala Gln Ala Ser Ala Thr Pro Ala Ser Gly Gly Ala
   385                 390                 395                 400
   Ser Thr Gly Asn Leu Val Val Gln Tyr Lys Val Gly Asp Thr Ser Ala
                   405                 410                 415
   Thr Asp Asn Gln Met Lys Pro Ser Phe Asn Ile Lys Asn Asn Gly Thr
                   420                 425                 430
   Thr Pro Val Asn Leu Ser Gly Leu Lys Leu Arg Tyr Tyr Phe Thr Lys
               435                 440                 445
   Asp Gly Thr Ala Asp Met Ser Ala Ser Phe Asp Trp Ala Gln Ile Gly
       450                 455                 460
   Ala Ser Asn Val Ser Ala Ala Phe Ala Asn Phe Thr Gly Ser Asn Thr
   465                 470                 475                 480
   Asp Thr Tyr Val Glu Leu Ser Phe Ser Ala Gly Ser Gly Ser Ile Pro
                   485                 490                 495
   Ala Gly Gly Gln Thr Gly Asp Ile Gln Leu Arg Met Tyr Lys Thr Asp
                   500                 505                 510
   Trp Ser Asn Phe Asn Glu Ala Asn Asp Tyr Ser Tyr Asp Gly Ala Lys
               515                 520                 525
   Thr Ala Tyr Ala Asp Trp Asn Arg Val Thr Leu His Gln Asn Gly Thr
       530                 535                 540
   Leu Val Trp Gly Thr Thr Pro
   545                 550